# EUROPEAN PATENT SPECIFICATION

(11) **EP 3 875 024 B1**
(45) Date of publication and mention of the grant of the patent: **26.02.2025**
(21) Application number: 21160470.7
(22) Date of filing: 03.03.2021
(51) Int. Cl.: A61B 3/00, A61B 3/18

(54) **EYE EXAMINATION SYSTEM CONTROL PROGRAM**
STEUERPROGRAMM FÜR EIN AUGENUNTERSUCHUNGSSYSTEM
PROGRAMME DE CONTRÔLE DU SYSTÈME D'EXAMEN DES YEUX

(30) Priority: 04.03.2020 JP 2020037330
(43) Date of publication of application: 08.09.2021
(73) Proprietor: NIDEK CO., LTD., Gamagori, Aichi (JP)
(72) Inventor: AOKI, Kenji, Gamagori, Aichi (JP)
(74) Representative: Hoefer & Partner Patentanwälte mbB

(56) References cited:
- EP-A1- 3 092 941
- EP-A2- 2 005 880
- WO-A1-2010/125908
- WO-A1-2020/105047
- JP-A- 2004 033 275
- JP-A- 2007 282 672
- US-A1- 2019 150 735

## Description

### TECHNICAL FIELD

The present disclosure relates to an eye examination system control program.

### BACKGROUND

A stationary eye examination device is used by being placed on an eye examination table (hereinafter, simply referred to as a table). At the time of examination, it is necessary to match the height of an examination axis (such as an imaging optical axis and a measurement optical axis) of the eye examination device and a subject eye. Therefore, after adjusting the height of the table in accordance with the sitting height of the examination subject, the height of the eye examination device is adjusted.

As an example, JP-A-2004-33275 discloses a technique for repeating a setting of the height of a table or the like in a past examination stored in association with identification data of an examination subject in a case of performing a new examination.

US 2019/150735 A1 discloses an eye examination system control program for controlling an eye examination system, wherein the eye examination system comprises at least first and second separate eye examination units, wherein each eye examination unit separately comprises an eye examination device, and wherein the eye examination system control program when executed by a control computer in the eye examination system causes execution of: an acquisition step of acquiring height information indicating a height of the examination axis in a case where the first eye examination unit of the eye examination units measures a subject eye, and a height adjustment step of controlling the height adjuster in the second eye examination unit of the eye examination units, based on the height information and a difference in eye level of the eye examination devices between the first eye examination unit and the second eye examination unit to adjust a height of the examination axis in the second eye examination unit. This document discloses also a table on which the eye examination device is placed, and a height adjuster for adjusting a height of at least one of the eye examination device and the table to match heights of a subject eye and an examination axis of the eye examination device.

By the way, there are various types of stationary eye examination devices depending on an application thereof, and a plurality of types of eye examination devices are adopted in one facility. Since there are common elements such as a base and a face support portion among different models, attempts are being made to unify (commonize) configurations such as shape and dimensions between models.

However, since a size and weight of an optical system or the like, which are necessary for examination, are widely different between models, it is difficult to unify the size and the weight for each model. As a result, it was not practical to completely unify eye level, which is the height from a bottom surface of an eye examination device to an examination axis, among the different models.

### SUMMARY

An object of the present disclosure is to provide an eye examination system control program in which a load for height adjustment is further reduced.

According to the invention, there is provided an eye examination system control program comprising the features of claim 1.

According to the present disclosure, the load for height adjustment is further reduced.

### BRIEF DESCRIPTION OF DRAWINGS

Fig. 1 is a diagram showing a schematic configuration of an eye examination system according to an example.
Fig. 2A is a diagram showing a first eye examination unit according to the example.
Fig. 2B is a diagram showing a second eye examination unit according to the example.
Fig. 3 is a flowchart for explaining an eye examination system control program according to the example.

### DETAILED DESCRIPTION

### <Overview>

Hereinafter, an embodiment in the present disclosure will be described. An eye examination system control program according to the embodiment is used for adjusting a height of an eye examination unit included in an eye examination system. The eye examination system control program is processing of reflecting a result of a height adjustment of a first eye examination unit, which is used first, on a second eye examination unit, in a case where the two examination units (the first eye examination unit and the second eye examination unit) examine the same examination subject (subject eye).

In the present embodiment, the eye examination system includes at least two separate eye examination units. Each eye examination unit includes an eye examination device, a table (eye examination table), and a height adjuster. The eye examination device may be a stationary device. The eye examination device included in the eye examination unit is placed on the table. Further, the height adjuster is used to match heights of the subject eye and an examination axis of the eye examination device. Specifically, the height adjuster adjusts the height of at least one of the eye examination device and the table. The height adjuster may be provided on each of the eye examination device and the table. In the following description, in particular, the height adjuster provided in the eye examination device is referred to as a device-side height adjuster, and the height adjuster provided in the table is referred to as a table-side height adjuster.

The eye examination system may also have a control computer. This control computer may be integrated with the eye examination device or the like, or may be a separate body. The eye examination system control program causes the eye examination system to perform at least an acquisition step and a height adjustment step by being executed by the control computer in the eye examination system.

In the acquisition step, height information indicating the height of the examination axis in a case where the first eye examination unit measures the subject eye is acquired.

In the height adjustment step, the height adjuster in the second eye examination unit is controlled based on the height information and a difference in eye level of the eye examination device between the first eye examination unit and the second eye examination unit. As a result, a height of an examination axis in the second eye examination unit is adjusted. At this time, the table-side height adjuster included in the second eye examination unit may be controlled based on the height information and the difference in eye level.

The eye level in the present embodiment is the height from the bottom surface of the eye examination device to the examination axis. The eye level is a fixed value. For example, in a device in which the height from the bottom surface to the examination axis can be adjusted by providing the device-side height adjuster, the eye level indicates a reference position in an adjustment range.

As described above, in the present embodiment, in the height adjustment in the second eye examination unit, in addition to the height information, the difference in eye level of the eye examination device between the first eye examination unit and the second eye examination unit are considered. Therefore, in the present embodiment, even in a case where the eye levels of the eye examination devices of the two eye examination units are different, the result of the height adjustment in the first eye examination unit can be preferably used in the height adjustment in the second eye examination unit. That is, even in a case where the same device did not perform the examination in the past, the height can be automatically adjusted, and the process becomes easy to smoothly proceed to the examination.

Further, for example, the height of the table in the examination unit (at least the table in the second eye examination unit) may be manually adjusted. In this case, the table may have a sensor (detector) for detecting the height of the table. In the height adjustment step, the height of the examination axis of the eye examination device may be adjusted by controlling the eye examination device of the second eye examination unit based on the difference in eye level, the height information, and a detection result of the sensor.

Since the height of the examination axis is adjusted on the device side in consideration of the result of manual height adjustment of the table, even in a case where the table is configured to adjust the height independently of the device in the second eye examination unit, the height of the examination axis can be adjusted appropriately.

### <About Eye Examination Unit Including Chair>

Further, in the present embodiment, each eye examination unit may further include a chair. In the height adjustment step, the height adjuster of the second eye examination unit may be controlled based on the difference in eye level between the two eye examination units, the difference in height of the chair between the two eye examination units, and the height information. As a result, even in a case where there is a difference in height of the chair between the two eye examination units, the height adjustment in the second eye examination unit can be preferably performed. The height of the chair may be a known value. Further, the chair whose height can be adjusted may have a sensor for detecting the height of the chair, and the value detected from the sensor may be used as the height of the chair in the height adjustment step.

Further, the chair may be provided with a drive portion that changes the height of the chair. The height adjuster of the eye examination system may adjust the height by driving the drive portion of the chair.

### <About Eye Examination Unit Including Jaw Rest>

In the present invention, the eye examination device of each eye examination unit includes a jaw rest. In the acquisition step, the jaw rest height information indicating a height of a jaw rest of the first eye examination unit is acquired. In the height adjustment step, based on the jaw rest height information, a height of the jaw rest of the second eye examination unit is adjusted. At this time, the jaw rest of the second eye examination unit may be controlled so that a distance from the eye level to an upper surface of the jaw rest matches between the two eye examination units.

### <Example>

Next, an example will be described based on the drawings.

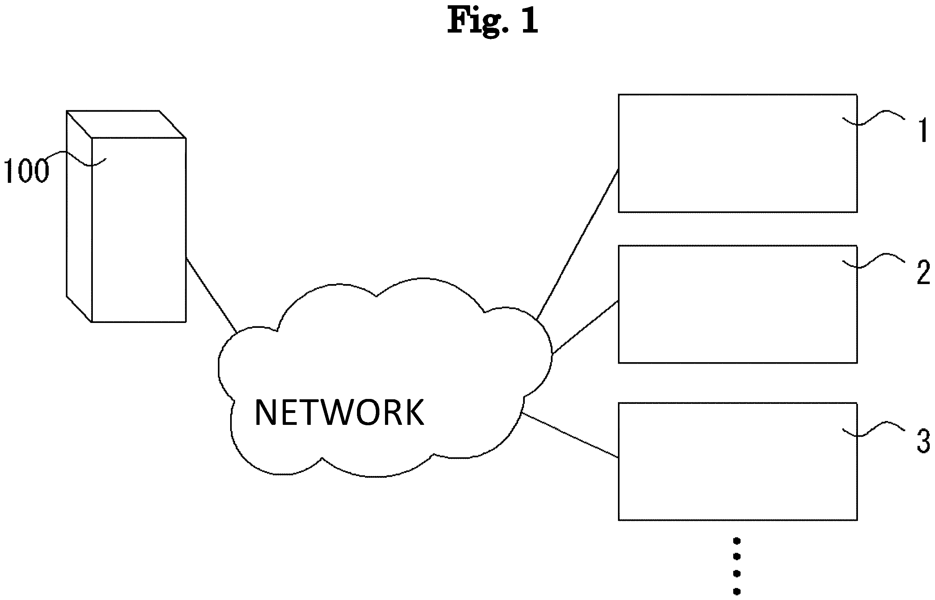

Fig. 1 shows a schematic configuration of an eye examination system in an example. In the eye examination system according to the example, a plurality of eye examination units 1, 2, 3, ..., and a network storage 100 are connected through a network.

Each of the eye examination units 1, 2, 3, ... includes at least an eye examination device and a table (eye examination table). In the eye examination units 1, 2, 3, ..., some eye examination units having different eye levels of the eye examination devices are mixed. The modality of the eye examination device may be different among the eye examination units 1, 2, 3, ....

The network storage 100 may store information used for examination performed by each eye examination unit 1, 2, 3, .... For example, in the present example, information indicating results of height adjustment in each of the examination units 1, 2, 3, ... is stored in association with identification information of each examination subject. Further, information indicating an eye level of the eye examination device in each of the examination units 1, 2, and 3 may be stored in the network storage 100. Further, in addition, past test results for each examination subject may be stored in the network storage 100.

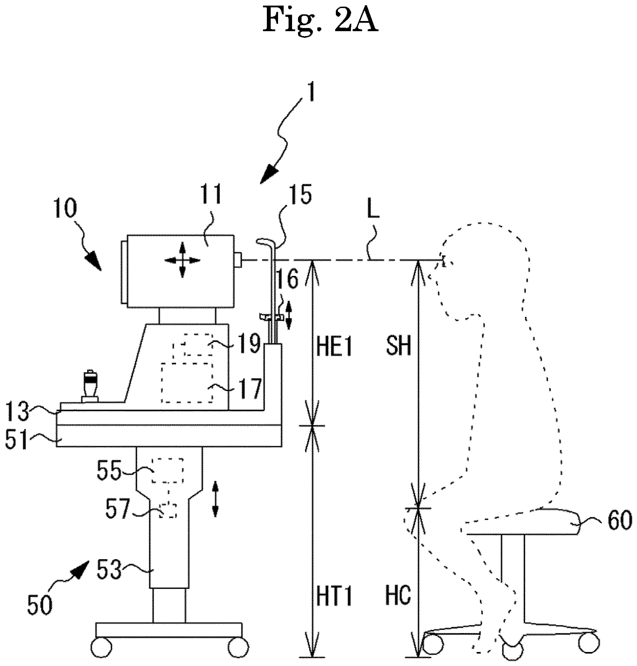

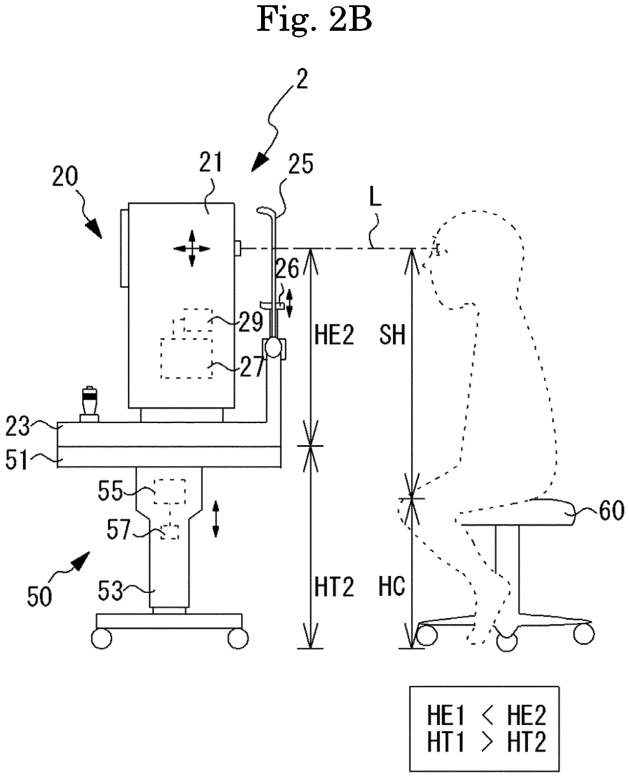

Next, detailed configurations of the eye examination units will be described with reference to Figs. 2A and 2B.

Fig. 2A shows a first eye examination unit 1, and Fig. 2B shows a second eye examination unit 2. Here, as an example, in the present example, a plurality of eye examination devices in each eye examination unit are classified into a first group and a second group based on a size and weight of an optical system and the like necessary for examinations. An eye examination device 10 of the first eye examination unit 1 illustrates a device of the first group, and an eye examination device 20 of the second eye examination unit 2 illustrates an eye examination device of the second group. A shape of a jaw rest, an eye level, and the like are unified among models classified into the same group, and are different between the first group and the second group. The first group is a device that can be relatively miniaturized. On the other hand, the second group is a relatively large device. For example, anterior eye segment examination devices (an objective eye refractive power measurement apparatus, a corneal endothelial cell imaging device, a tonometer, and the like) are classified into the first group, and fundus examination devices (an optical interference tomography, a fundus photography device, and the like) may be classified into the second group.

An axis indicated by a reference numeral L is an examination axis (an imaging optical axis, a measurement optical axis, or the like) in each of the eye examination units 1 and 2. For convenience of explanation, it is assumed that tables 50 included in the eye examination units 1 and 2 have the same configuration.

The first eye examination unit 1 has the eye examination device 10 and the table 50.

The eye examination device 10 includes an optical portion 11, a base 13, a face support portion 15, a drive portion 17, and a control portion 19. In the present example, the optical portion 11 includes a main optical system for examining a subject eye of an examination subject. The drive portion 17 three-dimensionally moves the optical portion 11. A vertical movement of the optical portion 11 by the drive portion 17 is used, for example, to match heights of the subject eye and the examination axis L. More specifically, the vertical movement may be used for fine adjustment.

Further, the face support portion 15 is provided with a jaw rest 16 that supports a jaw of the examination subject. A height of the jaw rest 16 is adjustable. In the present example, the jaw rest 16 is moved in a vertical direction by driving a jaw rest drive portion (not shown). The height of the jaw rest 16 is adjusted in accordance with a distance from the subject eye to the jaw.

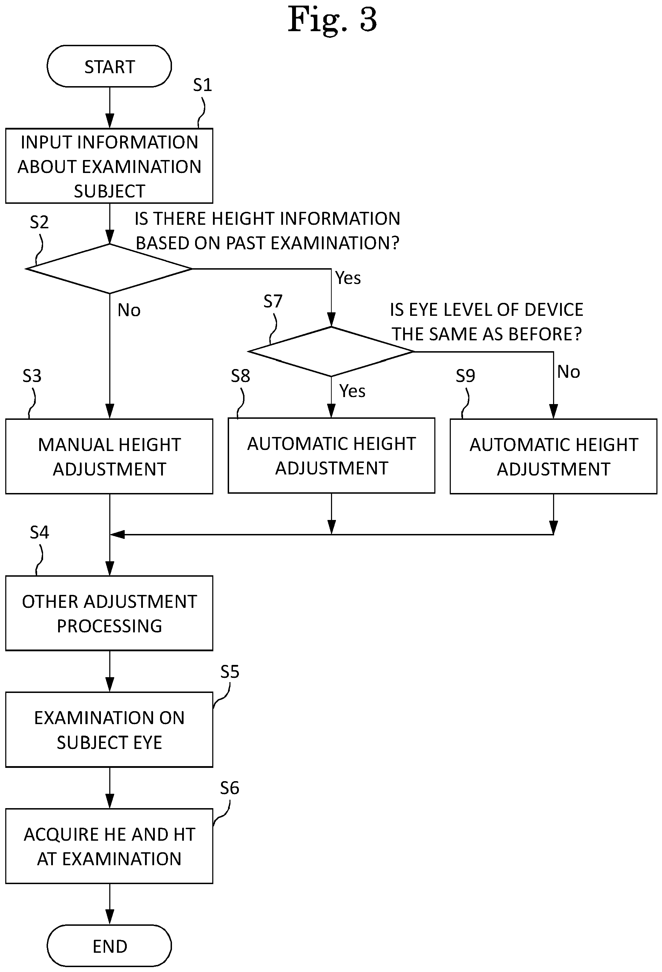

The control portion 19 controls each portion of the eye examination device 10. In the present example, the table 50 that supports the eye examination device 10 is also controlled based on the instruction from the control portion 19. In the present example, the control portion 19 executes the eye examination system control program, so that each processing shown in the flowchart of Fig. 3 is executed.

In the present example, the table 50 includes a top plate 51, a leg 53, a control portion 55, and a drive portion 57. As shown in Fig. 2A, a height of the top plate 51 is changed by the expansion and contraction of the leg 53 with the eye examination device 10 placed on the top plate 51. In the present example, the height of the top plate 51 is the height of the table 50.

The second eye examination unit 2 has the eye examination device 20 and the table 50. In the present example, an eye level of the eye examination device 20 in the second eye examination unit 2 is higher than an eye level of the eye examination device 10 in the first eye examination unit 1. The eye examination device 20 of the second eye examination unit 2 includes an optical portion 21, a base 23, a face support portion 25, a drive portion 27, and a control portion 29. For the detailed explanation of each portion, the description of each portion in the eye examination device 10 is incorporated, and the details are not repeated. The reference numeral 26 in the face support portion 25 is a jaw rest that can be driven in the vertical direction.

Here, as shown in Figs. 2A and 2B, the height of the examination axis L of the eye examination device 10 in the first eye examination unit 1 is represented by HE1, and the height of the table 50 is represented by HT1. Further, the height of the examination axis L of the eye examination device 20 in the second eye examination unit 2 is represented by HE2, and the height of the table 50 is represented by HT2. Further, the height of the chair is represented by HC, and the height from the chair to the subject eye is represented by SH. In the present example, the height of the chair is assumed to be constant for convenience of explanation.

In the first eye examination unit 1, by driving and controlling the drive portions 17 and 57 so that SH + HC = HE1 + HT1, the height of the examination axis L is adjusted to an appropriate position with respect to the subject eye. Similarly, in the second eye examination unit 2, by driving and controlling the drive portions 27 and 57 so that SH + HC = HE2 + HT2, the height of the examination axis L is adjusted to an appropriate position with respect to the subject eye. However, as described above, the eye level of the eye examination device 20 in the second eye examination unit 2 is higher than the eye level of the eye examination device 10 in the first eye examination unit 1. Therefore, in a case where each of the eye examination units 1 and 2 examines the same examination subject, the height from the bottom surface of each of the eye examination devices 10 and 20 to the examination axis L and the height of the table 50 may be different between the eye examination units.

As described above, according to the present example, the result of the height adjustment based on the past examination can be used for the height adjustment in the current examination even between the devices having different eye levels. That is, even in a case where the same device did not perform the examination in the past, the height can be automatically adjusted, and the process becomes easy to smoothly proceed to the examination.

### <Operation Explanation>

Next, the operation of the eye examination unit will be described with reference to the flowchart of Fig. 3. The flowchart of Fig. 3 shows the flow of the eye examination system control program in the example.

First, the information about the examination subject is input to the eye examination system (S1). At this time, information for specifying the examination subject (for example, identification information about the examination subject) may be input. In the present example, the information of the examination subject is input to the eye examination device included in the eye examination system.

Next, it is determined whether or not the height information based on the past examination corresponding to the examination subject to which the information is input is present in the eye examination device or the network storage (S2). The height information indicates the height of the examination axis in a case where the examination was performed by the eye examination system in the past. At this time, the height information may be based on the past examination in the eye examination unit used currently, or may be based on the past examination in another eye examination unit. The height information based on the past examination specifies the height of each of the eye examination device and the table in the eye examination unit used for the examination.

If there is no height information based on past examinations, manual height adjustment is required of an examiner (S3). At that time, a message that manual adjustment is necessary may be displayed, or may be announced by voice or the like. Further, in this case, the heights of the eye examination device and the table are manually adjusted in accordance with the position of the subject eye through an operation portion (not shown).

After adjustment, the examiner instructs the examination subject to place his or her jaw on the jaw rest. Other adjustment processing is performed while the face support portion including the jaw rest supports the examination subject's face (S4). At this time, for example, the jaw rest may be adjusted. Further, for example, alignment adjustment in the horizontal direction between the subject eye and the device may be performed. Further, the optical system built into the optical portion 11 may be driven for such focus adjustment. After the adjustment, the examination is executed (S5). Then, a test result is stored in a built-in memory of the eye examination device, the network storage 100, or the like in association with the information for specifying the examination subject. In addition, in the present example, the height information based on the current examination is also stored (S6).

Returning to S2, the description will continue. If the height information based on the past examination is present in the eye examination device or the network storage (S2: Yes), the determination processing of S7 is executed. That is, it is determined whether or not the eye level of the eye examination device in the eye examination unit used in the past examination is the same as the eye level of the eye examination device used currently (S7).

If the eye level of the eye examination device in the eye examination unit used in the past examination is the same as the eye level of the eye examination device used currently, the height information obtained in the past examination can be used as it is in the current examination. That is, in the present example, the height of the examination axis is adjusted so that the HTo and HEo specified from the height information based on the past examination are applied to the table and the eye examination device as they are (S8). After the adjustment, the processing of S4 to S6 is executed.

On the other hand, if the eye level of the eye examination device in the eye examination unit used in the past examination is different from the eye level of the eye examination device used currently (S7: No), the height adjustment is performed using the height information based on the past examination in consideration of the difference in eye level (S9). For example, if the eye level of the eye examination device used currently is a value higher than the eye level of the eye examination device used in the past, the table and the eye examination device may be controlled so that the height of the eye examination device and the height of the table are HT+a and HTo-a (a is the difference in eye level), respectively. After such adjustment, the processing of S4 to S6 is executed.

As described above, according to the present example, the height adjustment in the current examination can be satisfactorily performed, based on the height information obtained in the past examination, regardless of the difference in eye level of the eye examination device between the eye examination unit used in the past examination and the eye examination unit used in the current examination.

Although the above description has been made based on the embodiment, the present invention is not limited to the above embodiment, the invention being limited only by the scope of the appended claims.
1 first eye examination unit
2 second eye examination unit
10 eye examination device
20 eye examination device
17 drive portion
27 drive portion
50 table
57 drive portion
L examination axis

## Claims

1. An eye examination system control program for controlling an eye examination system, wherein:
- the eye examination system comprises at least first and second separate eye examination units (1, 2),
- each eye examination unit (1, 2) separately comprises (i) an eye examination device (10, 20), (ii) an eye examination table (50) on which the respective eye examination device (10, 20) is placed, and (iii) a height adjuster for adjusting a height of the respective eye examination device (10, 20) and of the respective table (50) to match heights of a subject eye and of an examination axis (L) of the respective eye examination device (10, 20),
- the eye examination device (10, 20) of each eye examination unit (1, 2) each comprises a jaw rest (16), and
- the eye examination system control program - when executed by a control computer in the eye examination system - causes execution of:
- an acquisition step of acquiring height information indicating a height of the examination axis (L) in a case where the first eye examination unit (1) of the eye examination units (1, 2) measures a subject eye, and
- a height adjustment step of controlling the height adjuster in the second eye examination unit (2) of the eye examination units (1, 2), based on the height information and a difference in eye level of the eye examination devices (10, 20) between the first eye examination unit (1) and the second eye examination unit (2) to adjust a height of the examination axis (L) in the second eye examination unit (2),
- in the acquisition step, jaw rest height information indicating a height of a jaw rest (16) of the first eye examination unit (1) is acquired, and
- in the height adjustment step, based on the jaw rest height information, a height of the jaw rest (16) of the second eye examination (2) unit is adjusted.

2. The eye examination system control program according to claim 1, wherein:
- each height adjuster comprises a device-side height adjuster for adjusting the height of the examination axis (L) of the respective eye examination device (10, 20) and a table-side height adjuster for adjusting a height of the respective eye examination table (50), and
- in the height adjustment step, at least the table-side height adjuster is controlled based on the difference in eye level and the height information.

3. The eye examination system control program according to claim 1 or 2, wherein:
- the table (50) in the second eye examination unit (2) has a detector for detecting a height of the table (50), and the height of the table (50) is manually adjustable, and
- in the height adjustment step, the height of the examination axis (L) in the eye examination device (20) of the second eye examination unit (1) is adjusted based on the difference in eye level, the height information, and a detection result of the detector.

4. The eye examination system control program according to any one of the preceding claims, wherein the height adjustment step is executed prior to position adjustment in a horizontal direction between the subject eye and the respective eye examination device (10, 20).

## Patentansprüche

1. Augenuntersuchungssystem-Steuerungsprogramm zum Steuern eines Augenuntersuchungssystems, wobei:
- das Augenuntersuchungssystem mindestens eine erste und eine zweite separate Augenuntersuchungseinheit (1, 2) umfasst,
- jede Augenuntersuchungseinheit (1, 2) jeweils (i) eine Augenuntersuchungsvorrichtung (10, 20), (ii) einen Augenuntersuchungstisch (50), auf dem die jeweilige Augenuntersuchungsvorrichtung (10, 20) platziert ist, und (iii) eine Höheneinstellvorrichtung zum Einstellen einer Höhe der jeweiligen Augenuntersuchungseinrichtung (10, 20) und des jeweiligen Tisches (50), um Höhen eines Subjektauges und einer Untersuchungsachse (L) der jeweiligen Augenuntersuchungseinrichtung (10, 20) aneinander anzupassen,
- die Augenuntersuchungseinrichtung (10, 20) jeder Augenuntersuchungseinheit (1, 2) jeweils eine Kieferstütze (16) umfasst und
- das Augenuntersuchungssystem-Steuerungsprogramm - wenn es von einem Steuercomputer in dem Augenuntersuchungssystem ausgeführt wird - Ausführen von Folgenden veranlasst:
- einem Erfassungsschritt zum Erhalten von Höheninformationen, die eine Höhe der Untersuchungsachse (L) in einem Fall angeben, in dem die erste Augenuntersuchungseinheit (1) der Augenuntersuchungseinheiten (1, 2) ein Subjektauge misst, und
- einen Höheneinstellungsschritt zum Steuern der Höheneinstellvorrichtung in der zweiten Augenuntersuchungseinheit (2) der Augenuntersuchungseinheiten (1, 2), basierend auf den Höheninformationen und einer Differenz in Augenniveau der Augenuntersuchungsvorrichtungen (10, 20) zwischen der ersten Augenuntersuchungseinheit (1) und der zweiten Augenuntersuchungseinheit (2), um eine Höhe der Untersuchungsachse (L) in der zweiten Augenuntersuchungseinheit (2) einzustellen,
- im Erfassungsschritt Kieferstützenhöheninformationen erhalten, die eine Höhe einer Kieferstütze (16) der ersten Augenuntersuchungseinheit (1) angeben werden, und
- im Höheneinstellungsschritt basierend auf den Kieferstützenhöheninformationen eine Höhe der Kieferstütze (16) der zweiten Augenuntersuchungseinheit (2) eingestellt wird.

2. Augenuntersuchungssystem-Steuerungsprogramm nach Anspruch 1, wobei
- jede Höheneinstellvorrichtung eine vorrichtungsseitige Höheneinstellvorrichtung zum Einstellen der Höhe der Untersuchungsachse (L) der jeweiligen Augenuntersuchungsvorrichtung (10, 20) und eine tischseitige Höheneinstellvorrichtung zum Einstellen einer Höhe des jeweiligen Augenuntersuchungstisches (50) umfasst und
- im Höheneinstellungsschritt zumindest die tischseitige Höheneinstellvorrichtung basierend auf dem Unterschied in Augenniveau und den Höheninformationen gesteuert wird.

3. Augenuntersuchungssystem-Steuerungsprogramm nach Anspruch 1 oder 2, wobei:
- der Tisch (50) in der zweiten Augenuntersuchungseinheit (2) einen Detektor zum Erfassen einer Höhe des Tisches (50) umfasst und die Höhe des Tisches (50) manuell einstellbar ist, und
- im Höheneinstellungsschritt die Höhe der Untersuchungsachse (L) in der Augenuntersuchungsvorrichtung (20) der zweiten Augenuntersuchungseinheit (1) basierend auf dem Unterschied in Augenniveau, den Höheninformationen und einem Erfassungsergebnis des Detektors eingestellt wird.

4. Augenuntersuchungssystem-Steuerungsprogramm nach einem der vorhergehenden Ansprüche, wobei der Höheneinstellungsschritt vor Positionsanpassung in einer horizontalen Richtung zwischen dem Subjektauge und der jeweiligen Augenuntersuchungsvorrichtung (10, 20) ausgeführt wird.

## Revendications

1. Programme de commande de système d'examen de la vue pour commander un système d'examen de la vue, dans lequel :
- le système d'examen de la vue comprend au moins des première et seconde unités d'examen de la vue (1, 2) distinctes,
- chaque unité d'examen de la vue (1, 2) comprend séparément (1) un dispositif d'examen de la vue (10, 20), (ii) une table d'examen de la vue (50) sur laquelle le dispositif d'examen de la vue (10, 20) respectif est placé, et (iii) un dispositif de réglage de hauteur pour régler une hauteur du dispositif d'examen de la vue (10, 20) respectif et de la table (50) respective pour correspondre aux hauteurs d'un œil du sujet et d'un axe d'examen (L) du dispositif d'examen de la vue (10, 20) respectif,
- le dispositif d'examen de la vue (10, 20) de chaque unité d'examen de la vue (1, 2) comprend un support de mâchoire (16), et
- le programme de commande de système d'examen de la vue - lorsqu'il est exécuté par un ordinateur de commande dans le système d'examen de la vue - provoque l'exécution de :
- une étape d'acquisition consistant à acquérir des informations de hauteur indiquant une hauteur de l'axe d'examen (L) dans un cas où la première unité d'examen de la vue (1) des unités d'examen de la vue (1, 2) mesure un œil du sujet, et
- une étape de réglage de hauteur consistant à commander le dispositif de réglage de hauteur dans la seconde unité d'examen de la vue (2) des unités d'examen de la vue (1, 2), sur la base des informations de hauteur et d'une différence de niveau des yeux des dispositifs d'examen de la vue (10, 20) entre la première unité d'examen de la vue (1) et la seconde unité d'examen de la vue (2) pour régler une hauteur de l'axe d'examen (L) dans la seconde unité d'examen de la vue (2),
- dans l'étape d'acquisition, des informations de hauteur de support de mâchoire indiquant une hauteur d'un support de mâchoire (16) de la première unité d'examen de la vue (1) sont acquises, et
- dans l'étape de réglage de hauteur, sur la base des informations de hauteur de support de mâchoire, une hauteur du support de mâchoire (16) de la seconde unité d'examen de la vue (2) est réglée.

2. Programme de commande de système d'examen de la vue selon la revendication 1, dans lequel :
- chaque dispositif de réglage de hauteur comprend un dispositif de réglage de hauteur côté dispositif pour régler la hauteur de l'axe d'examen (L) du dispositif d'examen de la vue (10, 20) respectif et un dispositif de réglage de hauteur côté table pour régler une hauteur de la table d'examen de la vue (50) respective, et
- dans l'étape de réglage de hauteur, au moins le dispositif de réglage de hauteur côté table est commandé sur la base de la différence de niveau des yeux et des informations de hauteur.

3. Programme de commande de système d'examen de la vue selon la revendication 1 ou 2, dans lequel :
- la table (50) dans la seconde unité d'examen de la vue (2) présente un détecteur pour détecter une hauteur de la table (50), et la hauteur de la table (50) est réglable manuellement, et
- dans l'étape de réglage de hauteur, la hauteur de l'axe d'examen (L) dans le dispositif d'examen de la vue (20) de la seconde unité d'examen de la vue (1) est réglée sur la base de la différence de niveau des yeux, des informations de hauteur et d'un résultat de détection du détecteur.

4. Programme de commande de système d'examen de la vue selon l'une quelconque des revendications précédentes, dans lequel l'étape de réglage de hauteur est exécutée avant le réglage de position dans une direction horizontale entre l'œil du sujet et le dispositif d'examen de la vue (10, 20) respectif.
